# EUROPEAN PATENT SPECIFICATION

(11) **EP 1 294 353 B1**
(45) Date of publication and mention of the grant of the patent: **22.03.2006**
(21) Application number: 01949380.8
(22) Date of filing: 05.06.2001
(51) Int. Cl.: A61Q 19/00, A61Q 19/08, A61K 8/49

(54) **USE OF AN (R)-ENANTIOMER OF LIPOIC ACID IN COSMETICS AND DERMATOLOGICALS**
VERWENDUNG VON EINEM LIPONSAÜREN -(R)-ENANTIOMEREN IN KOSMETIKA UND DERMATOLOGIE
UTILISATION DE (R)-ENANTIOMERE D'ACIDE LIPOIQUE DANS DES COMPOSITIONS COSMETIQUES ET DERMATOLOGIQUES

(30) Priority: 06.06.2000 DE 10027875; 06.06.2000 US 209632 P
(43) Date of publication of application: 26.03.2003
(73) Proprietor: BASF Aktiengesellschaft, 67056 Ludwigshafen (DE)
(72) Inventor: STREICHER, Harald, 68161 Mannheim (DE); JENTZSCH, Axel, 68165 Mannheim (DE)
(74) Representative: Kinzebach, Werner
(86) International application number: PCT/EP2001/006385
(87) International publication number: WO 2001/093824

(56) References cited:
- EP-A- 0 572 922
- EP-A- 0 812 590
- WO-A-01/24795
- DE-A- 4 344 751
- DE-A- 4 417 038
- DE-A- 4 419 783

## Description

The present invention relates to the use of lipoic acid, lipoic acid derivatives and salts thereof having an (R)-enantiomer excess in cosmetic compositions for rejuvenating and /or revitalizing the skin.

The human skin is subject to ageing processes, it being possible to differentiate between intrinsic processes (chronoeageing) and exogenous factors. In addition, temporary or permanent changes in the appearance of the skin, for example, also arise, such as acne, greasy or dry skin, keratoses, rosaceae, light-sensitive, inflammatory, erythematous, allergic or autoimmune reactions, such as dermatoses, photodermatoses and others, the exact causes of which, and factors which influence them, are often not fully understood.

Exogenous factors include, for example, sunlight or artificial radiation sources having a comparable spectrum, and compounds which may form as a result of the radiation, such as undefined reactive, e.g. free radical or ionic, photoproducts. These factors also, however, include harmful or reactive compounds such as ozone, free radicals, for example the hydroxyl radical, singlet oxygen and other reactive oxygen or nitrogen compounds, cigarette smoke, natural and synthetic toxins, and others, which impair the natural physiology or morphology of the skin. The effect of these factors results, for example, in direct damage to the DNA of skin cells and the collagen, elastin or glycosamino glycan molecules of the extracellular matrix which are responsible for the strength of the skin. In addition, the harmful effects can, however, also lead to damage to the cells of the skin itself. A consequence of this is, for example, that the regenerability of the skin is reduced. A further consequence may be inflammatory reactions, in which case immunoregulatory compounds, such as interleukines, prostaglandines and histamines, inter alia, play a role.

The consequences of ageing are thinning of the skin, weaker meshing of epidermis and dermis, and a reduction in cell number and in supplying blood vessels. The ageing processes lead to the formation of fine lines and wrinkles, the skin becomes leathery and yellowish and starts to sag, and pigment disturbances arise.

It is known that exposure to sunlight leads to a large number of undesired effects in the skin: erythema, photosensitization and immunological changes are examples of predominantly acute reactions, while photoageing and carcinogenesis are long-term effects. In this connection, oxidative processes also appear to play a role in photoageing. Furthermore, it is known that, following irradiation of the skin, matrix-destroying enzymes are induced and that this induction can be reduced by low molecular weight compounds, such as, for example, retinoic acids. A further effect of UV radiation is the appearance of sunburn cells in the skin. In this connection, it is possible for necrotic processes to arise, which induce or intensify an inflammatory reaction.

The same factors also act on hair, likewise leading to possible damage. Hair becomes brittle, less elastic and dull; the surface structure of the hair is damaged.

The properties of cosmetic or dermatological compositions are therefore subject to a large number of requirements. Thus, for example, they should, inter aila, have a free-radical scavenging action, an antioxidative action, an antiinflammatory action or moisturizing action, should prevent or reduce the activity of the matrix-destroying enzymes, and/or regulate the further synthesis of collagen, elastin and/or proteoglycans.

It is known to use antioxidative compounds in dermatological or cosmetic preparations for protecting against decay. Moreover, they can, however, also be used in order to reduce harmful or undesired oxidative processes which proceed in human or animal skin. The skin is permanently exposed to oxidative stress, some of which originates from the external surroundings of the skin, but some of which is also based on endogenous factors. The skin's own protective mechanisms are generally insufficient to completely prevent oxidative processes in the skin, meaning that these processes presumably make a significant contribution to skin ageing, but also to general or medically abnormal changes in the skin.

DE-A-197 39 349 proposes adding antioxidative constituents to cosmetic and dermatological preparations to aid the endogenous protective mechanisms of the skin. However, the effect achieved is still in need of improvement.

α-Lipoic acid (1,2-dithiacyclopentane-3-valeric acid) serves as coenzyme in the oxidative decarboxylation of α-keto acids and can be found in the form of its (R)-enantiomer in virtually every cell of vegetable and animal organisms.

Antiphlogistic, analgesic and cytoprotective properties and its antioxidative action make lipoic acid an interesting active ingredient for pharmacy, cosmetics, food science and related fields (Biothiols in Health and Disease, editor Packer L. and Cadenas E., Marcel Dekker Inc., New York, Basel, Hongkong). For example, in Pharmacology Biochemistry and Behavior, Vol. 46, pp. 799-805 (1993) and in Ann. NY Acad. Sci., Vol. 717, pp. 122-128 (1994), Stoll et al. reported that lipoic acid can improve the long-term memory of old mice or the cognitive capability of rodents. In FASEB Journal, Vol. 13, pp. 411-418 (1999) , T.M. Hagen et al. describe a revitalizing effect of orally administered lipoic acid on old rats.

EP-A-0 572 922 describes the use of α-lipoic acid and derivatives thereof in combination with a vitamin for the preparation of medicaments having analgesic, antiphlogistic, antidiabetic, cytoprotective, antiulcerative, antinecrotic, neuroprotective, detoxifying, antiischemic, liver function-regulating, antiallergic, immunostimulating and antioncogenic action. In this connection, very general reference is made to the fact that for a number of these areas of application, an optically pure isomer can be more effective than the racemate.

EP-A-0 427 247 describes medicaments and methods for their preparation, which comprise R-α-lipoic acid or S-α-lipoic acid or the pharmaceutically suitable salts thereof. The medicaments have a cytoprotective action and are suitable for controlling painful and inflammatory disorders.

WO-A-97/10808 describes a method of treating or preventing skin damage, in particular as a result of inflammation and ageing, where a topical composition which comprises lipoic acid or a derivative thereof is applied to the damaged areas of skin. The use of lipoic acid and lipoic acid derivatives with an (R)-enantiomer excess is not described. In addition, the described compositions are still in need of improvement with regard to their action, in particular in the case of repairing skin damage which already exists.

It is an object of the present invention to provide an active ingredient for use in skin or hair cosmetics or for the preparation of dermatologicals. These cosmetics and dermatologicals should preferably prevent skin and hair damage and/or undesired changes in the appearance of the skin. In particular, they should be suitable for the treatment of skin and hair damage or undesired changes in the appearance of the skin which already exist.

We have found that this object is achieved, surprisingly, by the use of lipoic acid, lipoic acid derivatives or salts thereof with an (R)-enantiomer excess, which opens up an effective method of preventing and treating such disorders.

The invention therefore relates to the use of lipoic acid, lipoic acid derivatives or cosmetically acceptable salts thereof, in each case having an (R)-enantiomer excess (ee) of at least 40%, in cosmetic compositions for rejuvenating and/or revitalizing the skin.

The term "lipoic acid" refers according to the invention to 5-(1,2-dithiolan-3-yl)valeric acid, also called thioctic acid, thioctanic [sic] acid or thioctinic acid, of the formula I

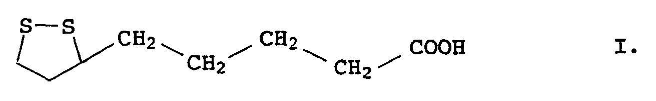

According to the invention, (R)-5-(1,2-dithiolan-3-yl)valeric acid of the formula II 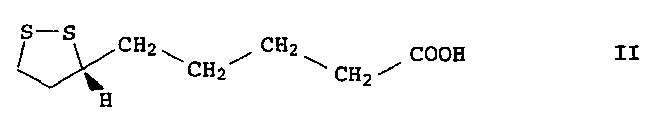 or derivatives or salts thereof is used in pure form or in the form of mixtures of optical isomers having an (R)-enantiomer excess (ee) of at least 40%.

The enantiomer excess (ee) is given by the following formula: ee[%] = (R-S)/(R+S) x 100. R and S are the descriptors of the CIP system for the two enantiomers and give the absolute configuration on the asymmetrical C(5) atom. The enantiomerically pure compound (ee = 100%) is also referred to as a homochiral compound.

It is also possible to use mixtures of lipoic acid, at least one derivative and/or at least one salt, provided an (R)-enantiomer excess of at least 40% is achieved.

The (R)-enantiomer excess is preferably at least 80%, in particular at least 98%.

Lipoic acid derivatives include in particular metabolites of lipoic acid, i.e. especially dihydrolipoic acid. Examples of further metabolites are lipoamide, lipoyllysine, di-6,8-bisnorlipoic acid and tetranorlipoic acid. The statements relating to lipoic acid also cover the respective optical isomers of the derivatives.

The cosmetically and pharmaceutically acceptable salts of lipoic acid or lipoic acid derivatives are preferably base addition salts in the present case.

The base addition salts include salts of lipoic acid or lipoic acid derivatives with inorganic bases, for example metal hydroxides or carbonates of alkali metal, alkaline earth metal or transition metals, or with organic bases, for example basic amino acids, such as arginine and lysine, amines of the formula NR³R⁴R⁵, in which the radicals R³, R⁴ and R⁵ may be identical or different and are hydrogen or optionally hydroxyl-substituted C₁-C₄-alkyl, in particular methyl, ethyl, propyl, isopropyl, butyl, isobutyl, sec-butyl or tert-butyl or mono- and diethanolamine, 1-amino-2-propanol or 3-amino-1-propanol, C₂-C₆-alkylenediamines, such as ethylenediamine or hexamethylenetetraamine, saturated cyclic amines having 4 to 6 ring carbon atoms, such as piperidine, piperazine, pyrrolidine and morpholine, and further organic bases, for example N-methylglucamine, creatine and tromethamine.

Preference is given to salts with inorganic bases, e.g. Na-, K-, Mg-, Ca-, Zn-, Cr- and Fe salts.

Lipoic acid and its derivatives and salts having the above-described optical purity are suitable in an advantageous manner for preventing and treating damage to keratinous surfaces, i.e. skin and skin appendages, such as, for example, hair, nails etc., of individuals, preferably mammals, in particular humans, useful animals or domestic animals. Use thereof can in cosmetic compostions such as body care compositions, decorative cosmetics etc., which are generally available without prescription.

The novel treatment of the affected skin can be directed toward individual disorders (anomalies, or medically abnormal states), although, if desired, it is also possible to treat two or more anomalies, which may be causally related to one another, using a composition according to the invention which optionally comprises an active ingredient combination.

Cosmetic and dermatological preparations based on (R)-enantiomer-enriched or pure lipoic acid, derivatives and/or salts thereof offer effective protection against oxidative processes, against processes caused by radiation or reactive compounds, and against damage caused directly or indirectly by such processes. They are advantageously suitable for the treatment of cosmetic or dermatological changes in skin and hair, such as, for example, skin ageing, loss of skin moisture, loss of skin elasticity, the formation of lines or wrinkles or of pigment disturbances or age spots.

(R)-enantiomer-enriched or pure lipoic acid is particularly preferably suitable for rejuvenating and/or revitalizing the skin. Advantageously, energizing effects can also generally be observed. In particular, (R)-enantiomer-enriched or pure lipoic acid has a positive effect on the function of the mitochondria. surprisingly, not only is oxidative damage to the skin prevented in this way, but existing damage can, at least partially, be repaired. In this connection, improvements with regard to the moisture value and/or the elasticity of the skin are noted. The use of (R)-enantiomer-enriched lipoic acid increases the further synthesis of collagen and/or elastin. This generally leads to a, at least partial, smoothing of wrinkles, and the complexion becomes more radiant and fresher. Said advantageous effects are generally also accompanied by a positive subjective feeling of having overall "youthful" skin.

(R)-lipoic acid-enriched formulations are also advantageously suitable for the treatment, care and cleansing of the skin or hair and can be used as makeup products in cosmetics. They preferably comprise from 0.001% by weight to 30% by weight of the active ingredient. In this connection, the composition is governed, for example, by the penetration properties of the active substance through the Stratum corneum and its ability to form a depot in the skin.

According to a preferred embodiment, the novel use of (R)-enantiomer-enriched or pure lipoic acid, derivatives and/or salts thereof advantageously takes place by regular application, e.g. in the form of a cosmetic or dermatological preparation, over a period of time. This depends on the desired result, i.e. the period of time can extend over the lifetime of the user, preferably over a period of time up to three months, particularly preferably over a week to two months, if the aim is to build up a depot in the skin. For aftersun application, the period of use for the purposes of the invention is a single application, but preferably a period of time of at least one day, particularly preferably over three days to three months, particularly preferably over one to two weeks.

For the purposes of the invention, it is advisable to topically apply the cosmetic or dermatological preparation of lipoic acid in an amount of from 0.1 µg/cm² to 2 mg/cm², between once per week and 4 to 5 times daily, preferably 3 times per week to 3 times daily, particularly preferably once to twice daily. Active ingredient amounts and proportions are based on the active ingredient, meaning that for salts and derivatives, appropriate conversion is necessary.

Those administration forms of lipoic acid and optionally additional active ingredients which are intended for aftersun applications advantageously have penetration properties which permit rapid penetration of the substance into the skin. By contrast, for applications with a "preconditioning" character, rapid penetration is generally unimportant, whereas the ability to build up a depot in the skin is advantageous.

Surprisingly, in the case of the use according to the invention, effective treatment, but also the prevention of prematurely aged skin (e.g. wrinkles, age spots, telangiectases, pigment disturbances) and/or prematurely aged skin appendages, radiation-induced skin damage or radiation-induced negative changes in the skin and/or skin appendages, environmentally-induced (ozone, free radicals, singlet oxygen, reactive oxygen or nitrogen compounds, cigarette smoke, toxins) skin damage or environmentally-induced negative changes in the skin and/or skin appendages, light-sensitive, inflammatory, erythematous, allergic or autoimmune changes in the skin and/or the skin appendages (in particular acne, greasy or dry skin, keratoses, rosaceae, dermatoses, atopic eczema, seborrheic eczema, photodermatoses, polymorphous light dermatoses), deficient, sensitive or hypoactive states of the skin and/or skin appendages, itching, dry skin conditions and horny layer barrier disturbances and/or hair loss and reduced hair growth is possible.

The novel use of (R)-enantiomer-enriched or pure lipoic acid, derivatives and/or salts thereof in cosmetic and dermatological preparations also, however, in a surprising and unforeseen manner, serves to calm sensitive and irritated skin, regulate collagen, hyaluronic acid, and elastinsynthesis, stimulate DNA synthesis, in particular in the case of deficient or hypoactive skin states, regulate the transcription and translation of matrix-destroying enzymes, in particular of matrix metalloproteinases, increase cell renewal and regeneration of the skin, increase endogenous protection and repair mechanisms for DNA, lipids and/or proteins, and, for pre- and post-treatment in the case of surgical interventions, to counteract skin irritations in particular and to promote regeneration processes of injured skin.

For the use according to the invention, the cosmetic and dermatological preparations are applied to the skin and/or hair in a sufficient amount in the manner customary for cosmetics.

For example, the (R)-enantiomer-enriched or pure lipoic acid and/or a derivative or salt thereof is used in cosmetic compositions for cleansing the skin, such as bar soaps, toilet soaps, curd soaps, transparent soaps, luxury soaps, deodorizing soaps, cream soaps, baby soaps, skin protection soaps, abrasive soaps, syndets, liquid soaps, pasty soaps, soft soaps, washing pastes, liquid washing, showering and bath preparations, e.g. washing lotions, shower preparations, shower gels, foam baths, cream foam baths, oil baths, bath extracts, scrub preparations, in-situ products, shaving foams, shaving lotions, shaving creams.

It is further suitable for skin cosmetic preparations, such as W/O or O/W skin and body creams, day and night creams, eye creams, light-protection compositions, aftersun products, handcare products, face creams, multiple emulsions, gelees, microemulsions, liposome preparations, niosomene preparations, antiwrinkle creams, face oils, lipogels, sport gels, moisturizing creams, bleaching creams, vitamin creams, skin lotions, care lotions, ampoules, aftershave lotions, preshaves, moisturizing lotions, tanning lotions, cellulite creams, depigmentation compositions, massage preparations, body powders, face lotions, face masks, deodorants, antiperspirants, nose strips, antiacne compositions, repellants, shaving compositions, depilatories, personal hygiene compositions, foot care compositions, baby care compositions and others.

Lipoic acid and/or a derivative or salt thereof can also be used in cosmetic compositions for hair care, such as hair treatments, hair lotions, hair rinses, hair emulsions, split-end fluids, neutralizing agents for permanent waves, hot-oil treatment preparations, conditioners, setting lotions, shampoos, hair tints and colorants, hairsprays, blow-waving setting lotions, shine sprays, hair brillantines, hair styling products, hair tonics, alopecia care compositions and others.

Lipoic acid and/or a derivative or salt thereof are also suitable for use in cosmetic preparations for decorative cosmetics, for example as foundation, powders, blushers, eye shadows, kohl pencils, eyeliners, eye foundation cream, lipsticks, eyebrow pencils, contour pencils, concealing sticks, stage make-up, mascara, eyelash tinting and coloring, make-up removing products and others.

The cosmetic, hygiene, dermatological or pharmaceutical preparations can, depending on the field of use, be prepared as spray (pump spray or aerosol), foam, gel, gel spray, lotion, cream, mousse, ointment, suspensions or powders.

It is also advantageous to administer lipoic acid and/or a derivative or salt thereof optionally with other active ingredients in encapsulated form, e.g. as cellulose encapsulation, in gelatine, with polyamides, in niosomes, wax matrices, with cyclodextrins or liposomally encapsulated.

The preparations can comprise cosmetic auxilaries as are customarily used in such preparations, e.g. preservatives, bactericides, perfumes, antifoams, dyes, pigments, thickeners, surface-active substances, emulsifiers, emollients, reviving agents, fats, oils, waxes or other customary constituents of a cosmetic or dermatological formulation, such as alcohols, polyols, polymers, foam stabilizers, solubilizers, electroytes, organic acids, organic solvents or silicone derivatives.

In addition to said active ingredients, the preparations can comprise further compounds which have an antioxidative, free-radical scavenger, skin moisturizing or moisture-retaining, antierythematous, antiinflammatory or antiallergic action, in order to supplement or enhance the action thereof. In particular, these compounds can be chosen from the group of vitamins, plant extracts, α- and β-hydroxy acids, ceramides, antiinflammatory, antimicrobial or UV-filtering substances, and derivatives thereof and mixtures thereof.

The antioxidants are advantageously chosen from amino acids (e.g. glycine, histidine, tyrosine, tryptophan) and derivatives thereof, imidazoles (e.g urocaninic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, aurothioglucose, propylthiouracil and other thiols (e.g. thioredoxin, glutathione, cysteine, cystine, cystamine and the glycosyl, N-acetyl, methyl, ethyl, prophyl [sic], amyl, butyl and lauryl, palmitoyl, oleyl, γ-linoleyl, cholesteryl and glyceryl esters thereof) and salts thereof, dilauryl thiodipropionate, distearyl thiodipropionate, thiodiproptionic [sic] acid and derivatives thereof (esters, ethers, peptides, lipids, nucleotides, nucleosides and salts) and sulfoximine compounds (e.g. buthionine sulfoximines, homocysteine sulfoximine, buthionine sulfones, penta-, hexa, heptathionine sulfoximine) in very low tolerated doses (e.g. pmol to µmol/kg) , also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of benzoin resin, rutic acid and derivatives thereof, butylhydroxytoluene, butylhydroxyanisole, norihydroguaiacic [sic] acid, nordihydroguaiaretic acid, trihydroxybutyrophenone, uric acid and derivatives thereof, mannose and derivatives thereof, sesamol, sesamolin, zinc and derivatives thereof (e.g. ZnO, ZnSO₄), selenium and derivatives thereof (e.g. selenomethionine), stilbenes and derivatives thereof (e.g. stilbene oxide, trans-stilbene oxide) and derivatives (salts, esters, ethers, sugars, nucleotides, nucleosides, peptides and lipids) of said active ingredients which are suitable according to the invention.

The antioxidants can be used alone or in the form of mixtures. The use amount of antioxidants in the preparations, alone or in combination, is preferably 0.001 to 30% by weight, based on the total weight of the preparation.

The preparations preferably further comprise substances which absorb UV radiation in the UV-B and/or UV-A region. Suitable UV filters are, for example, 2,4,6-triaryl-1,3,5-triazines, in which the aryl groups can in each case carry at least one substituent which is preferably chosen from hydroxyl, alkoxy, specifically methoxy, alkoxycarbonyl, specifically methoxycarbonyl and ethoxycarbonyl, and mixtures thereof. Also suitable are 4-aminobenzoic esters, where the amino group can optionally be alkylated or alkoxylated. These include, for example, isooctyl N,N-dimethyl-4-aminobenzoate. Also suitable are 2-hydroxybenzoic esters, such as, for example, the isooctyl ester. Further suitable UV filters are 2,4,6-trianilino(o-carbo-2'-ethylhexyl--1'-oxy)-1,3,5-triazine, 3-imidazol-4-ylacrylic acid and its ethyl ester, menthyl o-aminobenzoate, glyceryl p-aminobenzoate, 2,2'-dihydroxy-4-methoxybenzophenone (dioxybenzone), 2-hydroxy-4-methoxy-4-methylbenzophenone (Mexenon® [sic]), triethanolamine salicylate, dimethoxyphenylglyoxalic acid, 3-(4'sulfo)benzylidenebornan-2-one [sic] and its salts, 2,2',4,4'-tetrahydroxybenzophenone, 2,2'-methylenebis[6(2H-benzotriazol-2-yl-4-(1,1,3,3,-tetramethylbutyl)phenol] [sic], 2,2'-(1,4-phenylene)-bis-1H-benzimidazole-4,6-disulfonic acid and its Na salt, 2,4-bis[4-(2-ethylhexyloxy)-2-hydroxy]phenyl-6-(4-methoxyphenyl)-(1,3,5)triazine, 3-(4-methylbenzylidene)-camphor, polyethoxyethyl 4-bis(polyethoxy)paraaminobenzoate, 2,4-dihydroxybenzophenone and/or 2,2'-dihydroxy-4,4'-dimethoxybenzophenone-5,5'-disodium sulfonate.

The list of said UV filters which can be used in combination with the novel active ingredient combinations is not of course intended to be limiting. The total amount of filter substances is usually 0.1% to 30% by weight, preferably 0.5 to 15% by weight, in particular 1 to 10% by weight, based on the total weight of the preparations, to provide cosmetic preparations which protect the skin from the entire range of ultraviolet radiation.

If a lipid phase is used, then this is preferably chosen from mineral oils, mineral waxes, branched and/or unbranched hydrocarbons and hydrocarbon waxes, triglycerides of saturated and/or unsaturated, branched and/or unbranched C₈-C₂₄-alkanecarboxylic acids; synthetic, semisynthetic or natural oils, such as olive oil, palm oil, almond oil or mixtures; oils, fats or waxes; esters of saturated and/or unsaturated, branched and/or unbranched C₃-C₃₀-alkanecarboxylic acids and saturated and/or unsaturated, branched and/or unbranched C₃-C₃₀-alcohols, esters of aromatic carboxylic acids and saturated and/or unsaturated, branched and/or unbranched C₃-C₃₀-alcohols, for example isopropyl myristate, isopropyl stearate, hexyldecyl stearate, oleyl oleate; synthetic, semisynthetic and natural mixtures of the abovementioned esters, such as jojoba oil, alcohol benzoates or silicones oils such as, for example, cyclomethicone, dimethylpolysiloxane, diethylpolysiloxane, octamethylcyclotetrasiloxane and mixtures thereof, or dialkyl ethers.

If an aqueous phase is used, then it optionally additionally comprises a water-miscible solvent, such as C₁- to C₁₀-, preferably C₁- to C₅-alcohols, -diols or -polyols, and ethers thereof, preferably ethanol, isopropanol, propylene glycol, glycerol, ethylene glycol monoethyl ether etc.

Suitable emulsifiers are preferably known W/O- and O/W-emulsifiers, such as polyglycerol esters, sorbitan esters or partially esterified glycerides.

Suitable solubilizers are, in particular, ethoxylated sorbitan esters, ethoxylated lanolin alcohols and ethoxylated castor oil.

Customary native and synthetic thickeners or gel formers in the formulations are crosslinked polyacrylic acids and derivatives thereof, polysaccharides, such as xanthan gum or alginates, carboxymethylcellulose or hydroxycarboxymethylcellulose, hydrocolloids, such as gum arabic, or motmorillonite [sic] minerals such as bentonites or fatty alcohols, polyvinyl alcohol and polyvinylpyrrolidone.

Suitable propellants for aerosols are the customary propellants, for example propane, butane, pentane, dimethyl ether and others.

The invention relates to the use as claimed in claims 1 to 3, in form of a cosmetic composition comprising
I) lipoic acid, lipoic acid derivatives or pharmaceutically acceptable salts thereof, in each case having an (R)-enantiomer excess (ee) of at least 40%,
II) optionally at least one further cosmetic active ingredient, and
III) a cosmetically acceptable carrier.

The compositions preferably comprise components I in an amount of from 0.001 to 30% by weight, in particular 0.01 to 25% by weight, based on the total weight of the composition.

With regard to suitable components II) and III), reference is made to that stated previously.

The present invention further relates to compositions in the form of a commercial pack having at least one composition based on
i) lipoic acid, lipoic acid derivatives or pharmaceutically acceptable salts thereof, in each case having an (R)-enantiomer excess (ee) of at least 40%,
ii) optionally at least one further active ingredient, and
iii) a formulation base,
optionally together with instructions for the therapeutic use of lipoic acid, lipoic acid derivatives or pharmaceutically acceptable salts thereof, in each case having an (R)-enantiomer excess (ee) of at least 40%.

It goes without saying that commercial packs according to the invention can also comprise further preparations, in particular active-ingredient-containing formulations, and comprehensive instructions which go beyond the content given above.

The invention is illustrated in more detail by reference to the nonlimiting examples below.

### Examples of cosmetic preparations

| Type of formulation | Area of use | Example No. |
|---|---|---|
| O/W Emulsion | Soft skin lotion | 1 |
| W/O Emulsion | Protective handcream | 2 |
| | Suncare lotion | 3 |
| Multiple emulsion | W/O/W-Emulsion | 4 |
| Microemulsion | Microemulsion | 5 |
| Hydrophilic gel | Liposome gel | 6 |
| | Blunted oil gel | 7 |
| Lipophilic gel | Oil gel | 8 |
| Stick formulation | Sun protection lipstick balm | 9 |
| Aqueous cosmetics | Cooling body splash | 10 |
| Decorative cosmetics | Foundation | 11 |
| | Liquid foundation | 12 |
| Oils | Suncare oil | 13 |
| Body cleansers | Deep face cleanser | 14 |
| Rinse-out hair after-treatments | Conditioner | 15 |
| Leave-in hair after-treatments | Hair wax | 16 |
| | Antidandruff hair tonic | 17 |
| Aerosol | Foot deodorant spray | 18 |
| | Hairspray | 19 |

### Example 1

**Soft skin fluid**

| | % by weight |
|---|---|
| Ceteareth-6 (reaction product of fatty alcohol and ethylene oxide) and stearyl alcohol | 2.50 |
| Ceteareth-25 (reaction product of fatty alcohol and ethylene oxide) | 2.50 |
| Hydrogenated coconut fatty glyderides [sic] | 1.50 |
| Polyethylene glycol (40 EO units)/ dodecyl glycol copolymer | 3.00 |
| Dimethicone | 3.00 |
| Phenethyldimethicone | 2.00 |
| Cyclomethicone | 1.00 |
| Cetearyl octanoate | 5.00 |
| Avocado oil | 1.00 |
| Sweet almond oil | 2.00 |
| Wheatgerm oil | 0.80 |
| Panthenol USP | 1.00 |
| Phytantriol | 0.20 |
| Tocopheryl acetate | 0.30 |
| Propylene glycol | 5.00 |
| Perfume | q.s. |
| Preservatives | q.s. |
| Lipoic acid (>98% ee) | q.s. |
| Water | 69.20 |

### Example 2

**Protective handcream**

| | % by weight |
|---|---|
| Cetearyl alcohol | 1.00 |
| Glyceryl stearate | 1.50 |
| Stearyl alcohol | 1.50 |
| Cetyl palmitate | 2.00 |
| Tocopheryl acetate | 0.50 |
| Dimethicone | 8.00 |
| Ceteareth-6 (reaction product of fatty alcohol and ethylene oxide) and stearyl alcohol | 3.00 |
| Octyl methoxycinnamate | 5.00 |
| Propylene glycol | 8.00 |
| Panthenol | 1.00 |
| Evening primrose oil | 3.00 |
| Ethoxylated hydrogenated castor oil (7 EO) | 6.00 |
| Glyceryl oleate | 1.00 |
| Phenethyldimethicone | 3.00 |
| Beeswax | 1.50 |
| Carob gum | 0.80 |
| Silk powder | 0.80 |
| Preservatives | q.s. |
| Perfume | q.s. |
| Borax | 0.10 |
| Lipoic acid (>98% ee) | q.s. |
| Water | 52.30 |

### Example 3

**Suncare lotion**

| | % by weight |
|---|---|
| Ethoxylated hydrogenated castor oil (7 EO) | 6.00 |
| Ethoxylated hydrogenated castor oil (40 EO) | 0.50 |
| Isopropyl palmitate | 7.00 |
| Polyethylene glycol (40 EO)/ dodecyl glycol copolymer | 2.00 |
| Jojoba oil | 3.00 |
| Magnesium stearate | 0.60 |
| Octyl methoxycinnamate | 8.00 |
| C₁₂₋₁₅-Alkyl benzoate | 5.00 |
| Titanium dioxide | 4.00 |
| Propylene glycol | 5.00 |
| EDTA | 0.20 |
| Preservatives | q.s. |
| Water | 57.20 |
| Sodium ascorbyl phosphate | 1.00 |
| Tocopheryl acetate | 0.50 |
| Lipoic acid (>98% ee) | q.s. |
| Perfume | q.s. |

### Example 4

**Multiple emulsion**

| | % by weight |
|---|---|
| Mineral oil | 7.50 |
| Cetearyl octanoate | 2.50 |
| Aluminum stearate | 0.25 |
| Magnesium stearate | 0.25 |
| Microcrystalline wax H | 0.50 |
| Cetearyl alcohol | 1.00 |
| Lanolin alcohol | 1.50 |
| Mineral alcohol and lanolin alcohol | 1.50 |
| Ethoxylated hydrogenated castor oil (7 EO) | 0.75 |
| Polyethylene glycol (40 EO)/dodecylglycol copolymer | 2.00 |
| Ceteareth-6 (reaction product of fatty alcohol and ethylene oxide) and stearyl alcohol | 2.00 |
| Ceteareth-25 (reaction product of fatty alcohol and ethylene oxide) | 2.00 |
| Trilauret-4 [sic] phosphate | 1.00 |
| Hydroxyethylcellulose | 0.20 |
| Propylene glycol | 7.50 |
| Magnesium sulfate | 0.25 |
| Lipoic acid (>98% ee) | q.s. |
| Water | 69.30 |

### Example 5

**Microemulsion**

| | % by weight |
|---|---|
| Ceteareth-25 (reaction product of fatty alcohol and ethylene oxide) | 13.00 |
| Glyceryl cocoate (7 EO) | 20.00 |
| Octyldodecanol | 5.00 |
| Preservatives | q.s. |
| Lipoic acid (>98% ee) | q.s. |
| Water | 62.00 |

### Example 6

**Liposome gel**

| | % by weight |
|---|---|
| Ethoxylated hydrogenated castor oil (40 EO) | 1.00 |
| Bisabolol rac. | 0.10 |
| Propylene glycol | 8.00 |
| Panthenol | 0.50 |
| Water and tocopheryl acetate and polysorbate 80 and caprylic/capric triglyceride and lecithin | 3.00 |
| Preservatives | q.s. |
| Perfume | q.s. |
| Carbomer | 0.50 |
| Lipoic acid (>98% ee) | q.s. |
| Triethanolamine | 0.70 |
| Water | 86.30 |

### Example 7

**Blunted oil gel**

| | % by weight |
|---|---|
| Silicon dioxide | 5.00 |
| Dimethicone | 10.00 |
| Cetearyl octanoate | 40.00 |
| Caprylic/capric triglyceride | 8.00 |
| Phenethyldimethicone | 2.00 |
| Mineral oil | 28.50 |
| Sweet almond oil | 5.00 |
| Phytantriol | 0.30 |
| Lipoic acid (>98% ee) | q.s. |
| Tocopherol | 0.50 |
| Perfume | 1.00 |

### Example 8

**Oil gel**

| | % by weight |
|---|---|
| Silicon dioxide | 5.00 |
| Dimethicone | 10.00 |
| Cetearyl octanoate | 30.00 |
| Caprylic/capric triglyceride | 10.00 |
| Isopropyl myristate | 5.00 |
| Phenethyldimethicone | 5.00 |
| Mineral oil | 28.20 |
| Jojoba oil | 5.00 |
| Phytantriol | 0.30 |
| Lipoic acid (>98% ee) | q.s. |
| Tocopherol | 0.50 |
| Perfume | 1.00 |

### Example 9

**Sun protection balm**

| | % by weight |
|---|---|
| Beeswax | 12.00 |
| Hydrogenated coconut fat glycerides | 5.00 |
| Castor oil | 40.00 |
| Isopropyl palmitate | 10.00 |
| Mineral oil | 10.00 |
| Candellila wax | 8.00 |
| Phenethyldimethicone | 5.00 |
| Lipoic acid (>98% ee) | q.s. |
| Petrolatum | 5.00 |
| 3-Benzophenone | 5.00 |

### Example 10

**Cooling body splash**

| | % by weight |
|---|---|
| Ethoxylated hydrogenated castor oil (40 EO) | 2.00 |
| Menthyl lactate | 0.20 |
| Alcohol | 5.00 |
| Glyceryl cocoate (7 EO) | 2.00 |
| Witch hazel | 5.00 |
| Allantoin | 0.10 |
| Bisabolol rac. | 0.20 |
| Propylene glycol | 5.00 |
| Panthenol USP | 0.50 |
| Lactic acid (80% strength) | 0.20 |
| Lipoic acid (>98% ee) | q.s. |
| Perfume | q.s. |
| Water | 79.80 |

### Example 11

**Foundation**

| | % by weight |
|---|---|
| Ceteareth-6 (reaction product of fatty alcohol and ethylene oxide) and stearyl alcohol | 9.00 |
| Dimethicone | 5.00 |
| Cetearyl octanoate | 8.00 |
| Macadamia nut oil | 5.00 |
| Propylene glycol | 5.00 |
| Water | 53.00 |
| Sicovit White E 171 | 8.00 |
| Sicomet Brown 70 13E 3717 | 2.00 |
| Lipoic acid (>98% ee) | q.s. |
| Perfume | q.s. |
| 3-Benzophenone | 5.00 |

### Example 12

**Liquid foundation**

| | % by weight |
|---|---|
| Ceteareth-6 (reaction product of fatty alcohol and ethylene oxide) and stearyl alcohol | 7.00 |
| Ceteareth-25 | 5.00 |
| Dimethicone | 5.00 |
| Cetearyl octanoate | 8.00 |
| Macadamia nut oil | 5.00 |
| Propylene glycol | 5.00 |
| Water | 53.00 |
| Sicovit White E 171 | 8.00 |
| Sicomet Brown 70 13E 3717 | 1.00 |
| Lipoic acid (>98% ee) | q.s. |
| Perfume | q.s. |
| 3-Benzophenone | 5.00 |

### Example 13

**Suncare oil**

| | % by weight |
|---|---|
| Cetearyl octanoate | 40.00 |
| Caprylic/capric triglycerides | 28.70 |
| Evening primrose oil | 3.00 |
| Macadamia nut oil | 5.00 |
| Isopropyl palmitate | 5.00 |
| Dimeticone [sic] | 3.00 |
| Octyl methoxycinnamate | 8.00 |
| Octocrylene | 5.00 |
| 3-Benzophenone | 2.00 |
| Phytantriol | 0.10 |
| Lipoic acid (>98% ee) | q.s. |
| Tocopheryl acetate | 0.20 |
| Perfume | q.s. |

### Example 14

**Deep face cleanser**

| | % by weight |
|---|---|
| Water | 65.60 |
| Cocoamidopropylbetaine | 5.00 |
| Sodium cocoate, hydrolyzed animal protein | 8.00 |
| Ethoxylated hydrogenated castor oil (40 EO) | 2.00 |
| Polyquaternium-44 | 7.70 |
| Bisabolol rac. | 0.20 |
| Panthenol | 1.00 |
| Perfume | 0.50 |
| Hydroxyethylcellulose | 2.00 |
| Lipoic acid (>98% ee) | q.s. |
| Propylene glycol | 5.00 |
| Jojoba wax | 3.00 |

### Example 15

**Conditioner**

| | % by weight |
|---|---|
| Ceteareth-6 and stearyl alcohol | 2.00 |
| Ceteareth-25 (reaction product of fatty alcohol and ethylene oxide) | 1.00 |
| Cetearyl octanoate | 6.00 |
| Ceteareth-3 | 2.00 |
| Cetearyl alcohol | 6.00 |
| Phytantriol | 1.00 |
| Propylene glycol | 5.00 |
| Polyquaternium-11 | 5.00 |
| Panthenol | 1.00 |
| Retinyl acetate | 0.50 |
| Perfume | q.s. |
| Lipoic acid (>98% ee) | q.s. |
| Preservatives | q.s. |
| Water | 70.50 |

### Example 16

**Hair wax**

| | % by weight |
|---|---|
| Polyethylene glycol-6 | 30.00 |
| Polyethylene glycol-75 | 45.00 |
| Liquid paraffin oil | 0.50 |
| Ethoxylated hydrogenated castor oil (40 EO) | 1.00 |
| Glycerol | 15.00 |
| 3-Benzophenone | 2.00 |
| Phytantriol | 0.10 |
| Lipoic acid (>98% ee) | q.s. |
| Perfume | q.s. |
| Water | 6.40 |

### Example 17

**Antidandruff hair tonic**

| | % by weight |
|---|---|
| Alcohol | 45.00 |
| Aloe vera (10-fold conc.) | 1.00 |
| Panthenol | 1.00 |
| Tocopheryl acetate | 0.50 |
| Ethoxylated hydrogenated castor oil (40 EO) | 0.50 |
| Allantoin | 0.10 |
| Hydrolyzed animal protein | 1.50 |
| 1-(4-Chlorophenoxy)-1-(1H-imidazolyl)-3,3-dimethyl-2-butanone | 0.30 |
| Perfume | 0.10 |
| Lipoic acid (>98% ee) | q.s. |
| Water | 50.00 |

### Example 18

**Foot deodorant spray**

| | % by weight |
|---|---|
| Ethoxylated hydrogenated castor oil (40 EO) | 0.80 |
| Alcohol | 20.00 |
| Farnesol | 0.12 |
| Menthyl lactate | 0.08 |
| 1,2 Propylene [sic] glycol | 3.20 |
| 4-Benzophenone | 1.20 |
| Glyceryl cocoate (7 EO) | 0.80 |
| Perfume | q.s. |
| Lipoic acid (>98% ee) | q.s. |
| Water | 13.80 |
| Butane | 60.00 |

### Example 19

**Hairspray**

| | % by weight |
|---|---|
| Aminomethylpropanol | 0.40 |
| Dimethicone copolyol | 0.030 |
| Alcohol | 43.67 |
| Pentane | 14.20 |
| Acrylate/acrylamide copolymer | 3.40 |
| Perfume | q.s. |
| Lipoic acid (>98% ee) | q.s. |
| Butanes [sic] | 2.40 |
| Isobutane | 35.90 |

## Claims

1. The use of lipoic acid, lipoic acid derivatives or cosmetically acceptable salts thereof, in each case having an (R)-enantiomer excess (ee) of at least 40%, in cosmetic compositions for rejuvenating and/or revitalizing the skin.

2. The use as claimed in claim 1, wherein energizing effects can be observed.

3. The use as claimed in any of the preceding claims, where the (R)-enantiomer excess is at least 80%, preferably at least 98%.

4. The use as claimed in any of the preceding claims in form of a cosmetic composition comprising
I) lipoic acid, lipoic acid derivatives or pharmaceutically acceptable salts thereof, in each case having an (R)-enantiomer excess (ee) of at least 40%,
II) optionally at least one further cosmetic active ingredient, and
III) a cosmetically acceptable carrier.

5. The use as claimed in claim 4, wherein the composition comprises component I in an amount of from 0.001 to 30% by weight, based on the total weight of the composition.

## Patentansprüche

1. Verwendung von Liponsäure, Liponsäurederivaten oder kosmetisch akzeptablen Salzen davon, jeweils mit einem (R)-Enantiomeren-Überschuss (ee) von mindestens 40 %, in kosmetischen Mitteln zur Verjüngung und/oder Revitalisierung der Haut.

2. Verwendung nach Anspruch 1, wobei energetisierende Effekte beobachtet werden können.

3. Verwendung nach einem der vorhergehenden Ansprüche, wobei der (R)-Enantiomeren-Überschuss mindestens 80 %, bevorzugt mindestens 98 % beträgt.

4. Verwendung nach einem der vorhergehenden Ansprüche in Form eines kosmetischen Mittels, enthaltend
I) Liponsäure, Liponsäurederivate oder pharmazeutisch akzeptable Salze davon, jeweils mit einem (R)-Enantiomeren-Überschuss (ee) von mindestens 40 %,
II) gegebenenfalls wenigstens einen weiteren kosmetischen Wirkstoff, und
III) einen kosmetisch akzeptablen Träger.

5. Verwendung nach Anspruch 4, wobei das Mittel die Komponente I in einer Menge von 0,001 bis 30 Gew.% bezogen auf das Gesamtgewicht des Mittels enthält.

## Revendications

1. Utilisation d'acide lipoïque, de dérivés d'acide lipoïque ou de leurs sels acceptables du point de vue cosmétique, ayant dans chaque cas un excès de (R)-énantiomère (ee) d'au moins 40 %, dans des compositions cosmétiques pour rajeunir et/ou revitaliser la peau.

2. Utilisation suivant la revendication 1, dans laquelle des effets énergétiques peuvent être observés.

3. Utilisation suivant l'une quelconque des revendications précédentes, où l'excès de (R)-énantiomère est d'au moins 80 %, de préférence d'au moins 98 %.

4. Utilisation suivant l'une quelconque des revendications précédentes, sous la forme d'une composition cosmétique comprenant
I) de l'acide lipoïque, des dérivés d'acide lipoïque ou leurs sels pharmaceutiquement acceptables, comportant dans chaque cas un excès de (R) -énantiomère (ee) d'au moins 40 %,
II) éventuellement au moins un autre ingrédient actif cosmétique, et
III) un support acceptable du point de vue cosmétique.

5. Utilisation suivant la revendication 4, dans laquelle la composition comprend le composant I en une quantité de 0,001 à 30 % en poids par rapport au poids total de la composition.
